# EUROPEAN PATENT SPECIFICATION

(11) **EP 2 630 953 B1**
(45) Date of publication and mention of the grant of the patent: **09.08.2017**
(21) Application number: 11854287.7
(22) Date of filing: 27.12.2011
(51) Int. Cl.: A61K 9/127, A61K 47/24, A61K 47/34, B01J 13/02

(54) **LIPOSOME COMPOSITION AND PROCESS FOR PRODUCTION THEREOF**
LIPOSOMZUSAMMENSETZUNG UND HERSTELLUNGSVERFAHREN DAFÜR
COMPOSITION DE LIPOSOMES ET SON PROCÉDÉ DE PRODUCTION

(30) Priority: 27.12.2010 JP 2010291110
(43) Date of publication of application: 28.08.2013
(73) Proprietor: Terumo Kabushiki Kaisha, Tokyo 151-0072 (JP)
(72) Inventor: YAMASHITA, Keiko, Ashigarakami-gun Kanagawa 259-0151 (JP); NOZAWA, Shigenori, Ashigarakami-gun Kanagawa 259-0151 (JP)
(74) Representative: Prüfer & Partner mbB Patentanwälte · Rechtsanwälte
(86) International application number: PCT/JP2011/080304
(87) International publication number: WO 2012/091054

(56) References cited:
- WO-A1-95/13796
- WO-A1-2008/050807
- WO-A1-2010/058840
- WO-A1-2012/058483
- WO-A2-2010/041255
- CN-A- 101 744 764
- JP-A- 7 173 052
- JP-A- 2009 132 629
- JP-A- 2010 260 828
- E. Kisak ET AL: "The Vesosome - A Multicompartment Drug Delivery Vehicle", Current medicinal chemistry, vol. 11, no. 2, 1 January 2004 (2004-01-01), pages 199-219, XP055090779, ISSN: 0929-8673, DOI: 10.2174/0929867043456197

## Description

### Technical Field

The present invention relates to a sustained-release liposome composition containing an effective component such as a drug.

### Background Art

Drugs needing frequent administration have a problem that frequent hospitalization and pains due to puncture or the like impose heavy burdens on the patient. In addition, it is difficult for patients suffering difficulty in swallowing to take a drug through the mouth, so that other administering method than peroral administration is demanded. Also, for patients needing a care giver such as patients of dementia, diseases of brain and Parkinson's disease, it is difficult to take the drug under the patients' own control; in these cases, therefore, such choices as other administering method than peroral administration or a treating method not needing frequent administration are demanded. Further, for patients whose daily living is hindered as soon as the drug stops working, such as patients of autonomic imbalance, a therapeutic method is demanded in which the drug's efficacy is not lost in a short time but can continue for a long period of time. Or, as for the pain after an operation, the patient has an attack of an unbearable pain as soon as the drug stops working, which may influence the rehabilitation and may cause leaving hospital to be postponed. Therefore, if the drug can remain showing its efficacy and suppressing pain for five to seven days after an operation, it is considered that the postoperative rehabilitation can be promoted, probably contributing to earlier leaving hospital.

From the foregoing, sustained release preparations by which a drug's efficacy can be maintained for a long time are highly expected as means for enhancing patients' QOL in all disease regions.

Most of the sustained release preparations which have been investigated heretofore are microspheres based on the use of polylactic acid-glycolic acid copolymer (PLGA). For instance, PLAG microspheres using donepezil hydrochloride, an effective ingredient of Aricept (registered trademark; Eisai Co., Ltd.) which is known as an Alzheimer type dementia treating agent, have been investigated and sustained release properties have been obtained therewith (Non-Patent Document 1). In the case of using PLGA, however, it is difficult to encapsulate, particularly, a water-soluble drug in high concentration and with high efficiency, and there are problems yet to be solved in order to attain a high drug encapsulation amount. In addition, the use of PLGA has a problem in that the use of an organic solvent in the preparation process makes indispensable the removal of the organic solvent (see, for example, Patent Documents 1 and 2) and that local intensification of acid attendant on decomposition of PLGA causes inflammation.

Other than the above, some approaches of sustained release preparations based on the use of a local anesthetic such as bupivacaine have also been investigated, but they have still a problem as to such retention properties that the pain tending to last for five to seven days after an operation can be suppressed for a sufficient period of time (Non-Patent Document 2). Besides, multivesicular liposome (MVL) has been developed as a lipid-based sustained release drug support for local or systemic drug delivery (Patent Documents 1 and 2). This approach, however, is also not yet satisfactory in regard to drug encapsulation amount and sustained release time.

### Prior Art Documents

### Patent Documents

Patent Document 1: JP-T-2001-505224
Patent Document 2: JP-T-2001-522870

### Non-patent Documents

Non-Patent Document 1: Biomaterials, 28 (2007), 1882-1888
Non-Patent Document 2: Anesthesiology, 101 (2004), 133-137

### Summary of Invention

### Technical Problem

It is an object of the present invention to provide a liposome composition in which a drug is moved from the outside into the inside along an ion gradient, into which the drug can thereby be introduced in a high encapsulation amount with high efficiency, and which has sustained release properties to such an extent that an effective concentration can be maintained at a clinically satisfactory level, and a process for production of the liposome composition.

### Technical Solution

The above problem can be solved by the present invention as follows.
(1) A liposome composition comprising:
   a first liposome having an outer membrane comprised of a multilayered lipid bilayer; and
   a plurality of second liposomes accommodated in a first liposome inner region defined by the outer membrane, the second liposomes each having an outer membrane comprised of a multilayered lipid bilayer,
   wherein the liposome composition has second liposome inner regions each defined by the outer membrane of each of the second liposomes, and
   an ion gradient is formed at least between each of the second liposome inner regions and the outside of the first liposome,
   wherein lipid membranes of the first liposome and the second liposomes are each comprised of lipid including phospholipid and cholesterol, and
   wherein the liposome composition is prepared as defined in claim 11.
(2) The liposome composition as described in the above paragraph (1), wherein the ion gradient is proton concentration gradient, and pH in the second liposome inner region or pH in the second liposome inner region and the first liposome inner region is lower than pH in the outside of the first liposome.
(3) The liposome composition as described in the above paragraph (1) or (2), wherein the first liposome has an average particle diameter within a range of 1 to 20 µm.
(4) The liposome composition as described in any of the above paragraphs (1) to (3), wherein a drug is contained in the second liposome inner region or in the second liposome and first liposome inner regions.
(5) The liposome composition as described in the above paragraph (4), wherein the drug is contained in a molar ratio (mol/mol) of not less than 0.05, based on total lipid.
(6) The liposome composition as described in any of the above paragraphs (1) to (4), wherein the drug to be encapsulated is capable of being encapsulated into liposomes by an ion gradient method, preferably is an ionizable amphipathic drug.
(7) The liposome composition as described in any of the above paragraphs (4) to (6), wherein the drug is a drug for treatment of cerebral vascular disorder, Parkinson's disease, dementia, analgesic agents, local anesthetics, and anti-malignancy agents.
(8) The liposome composition as described in any of the above paragraphs, wherein the phospholipid is a saturated phospholipid.
(9) The liposome composition as described in any of the above paragraphs for use in treatment by subcutaneous, intramuscular, intraperitoneal, intrathecal, extradural or intraventricular administration, preferably for subcutaneous administration.
(10) The liposome composition as described in any of the above paragraphs for use in treatment by being administered by use of a syringe or a spray-type device, or by an administration being carried out through a catheter inserted in a living body.
(11) A process for producing a liposome composition provided with an ion gradient between the inside and the outside of an outer membrane,
   the process comprising the steps of:
   mixing a first inner aqueous phase solution containing a compound for forming the ion gradient with a lipid-containing water-miscible solvent in a volume ratio of from 0.7 to 2.5 so as to prepare a first emulsion, the lipid including phospholipid and cholesterol;
   mixing a second inner aqueous phase solution with the first emulsion in a volume ratio of from 0.7 to 2.5 so as to prepare a second emulsion; and
   replacing an outer aqueous phase of the second emulsion with an aqueous solution which is lower than the first inner aqueous phase solution in the concentration of the compound for forming the ion gradient.
(12) The process for producing a liposome composition as described in the above paragraph (11), wherein the ion gradient is proton concentration gradient.
(13) The process for producing a liposome composition as described in the above paragraph (11) or (12), in which the first inner aqueous phase solution contains a sulfate, preferably wherein the sulfate is ammonium sulfate.
(14) The process for producing a liposome composition as described in any of the above paragraphs (11) to (13), further comprising
   a step of introducing a drug into the inside of the liposome composition by a driving force due to the ion gradient.

### Advantageous Effect

The present invention provides a liposome composition comprising:
a first liposome having an outer membrane comprised of a multilayered lipid bilayer; and
a plurality of second liposomes accommodated in a first liposome inner region defined by the outer membrane, the second liposomes each having an outer membrane comprised of a multilayered lipid bilayer,
wherein the liposome composition has second liposome inner regions each defined by the outer membrane of each of the second liposomes, and
an ion gradient is formed at least between each of the second liposome inner regions and the outside of the first liposome,
wherein lipid membranes of the first liposome and the second liposomes are each comprised of lipid including phospholipid and cholesterol, and
wherein the liposome composition is prepared as defined in claim 11. The liposome composition according to the present invention permits a drug to be encapsulated therein with high efficiency and is capable of long-time sustained release of the drug.

By the process for producing a liposome composition according to the present invention, it is possible to obtain a liposome composition which permits a drug to be encapsulated therein with high efficiency and which is capable of long-time sustained release of the drug.

### Brief Description of Drawings

[FIG. 1]

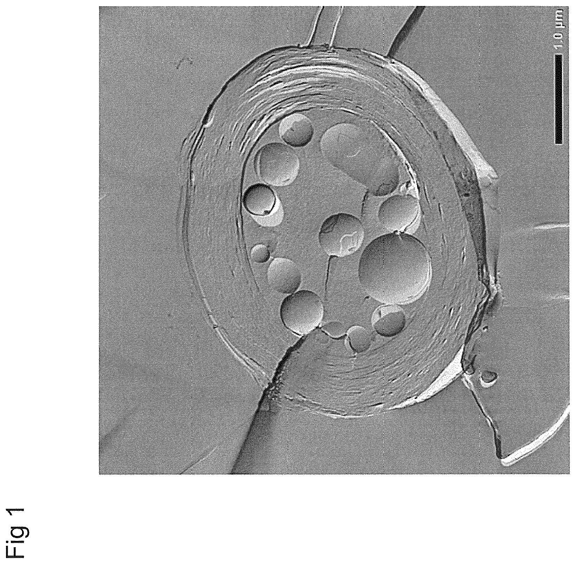

   FIG. 1 is a photograph (magnification: 32,000) obtained by transmission electron microscope (TEM) observation of a section of a liposome composition, after introduction of a drug, produced in Preparation Example 2 in an embodiment of the present invention.
[FIG. 2]

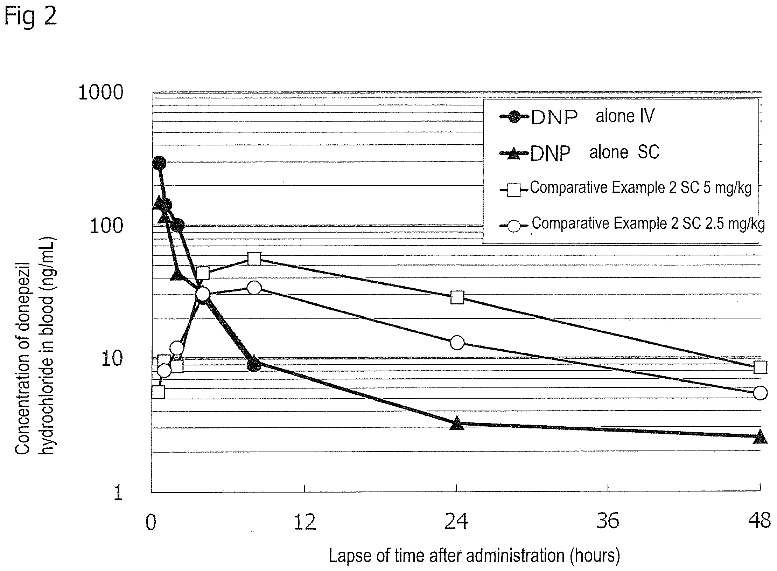

   FIG. 2 is a graph representing the results of pharmacokinetic profile, for example, donepezil liposome (Comparative Example 2) prepared by Extrusion Method-1.
[FIG. 3]

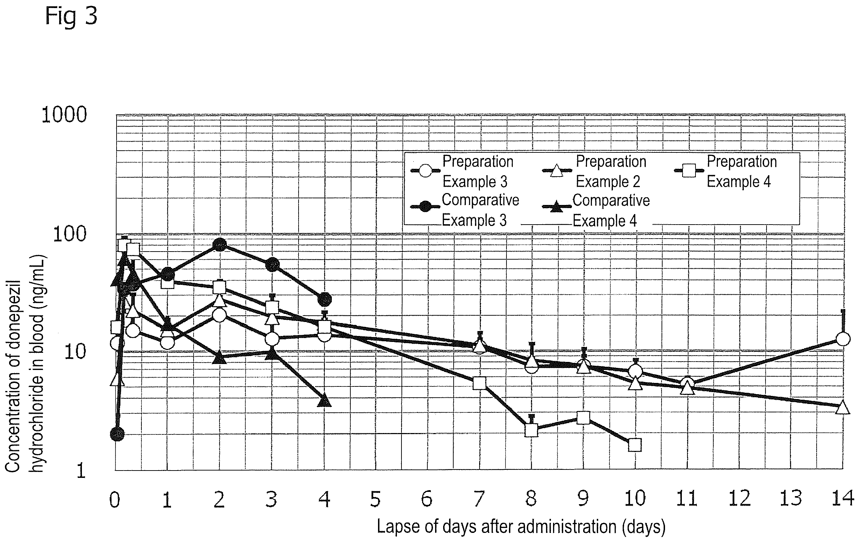

   FIG. 3 is a graph representing the results of pharmacokinetic profile, for example, liposome compositions obtained in Preparation Examples 2, 3 and 4 according to the present invention.
[FIG. 4]

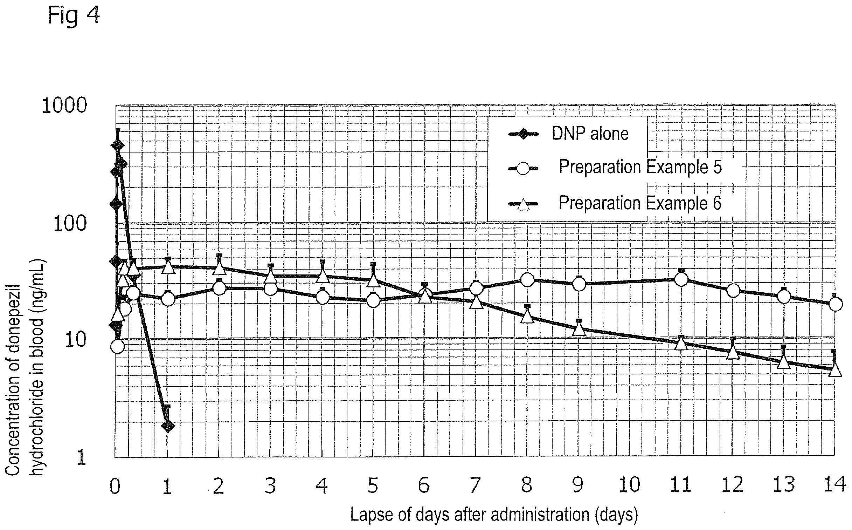

   FIG. 4 is a graph representing the results of pharmacokinetic profile, for example, liposome compositions obtained in Preparation Examples 5 and 6 according to the present invention.
[FIG. 5]

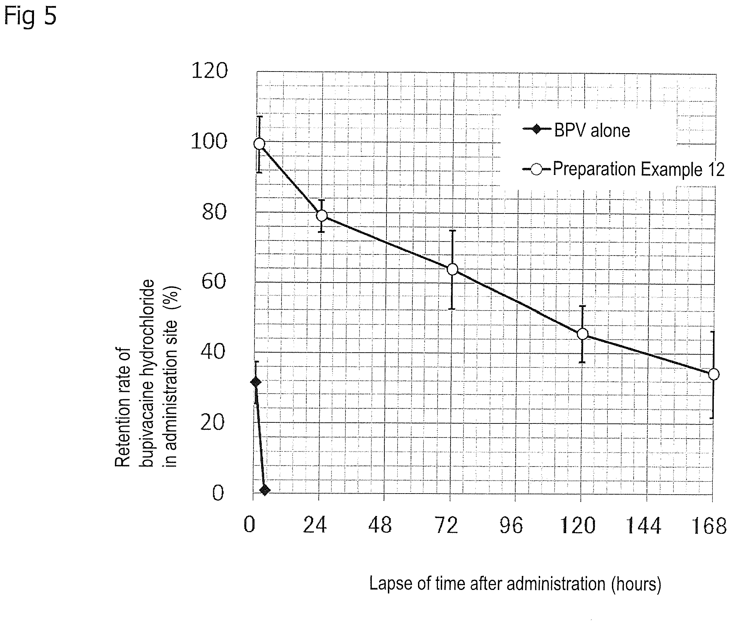

   FIG. 5 is a graph representing the results of pharmacokinetic profile of a liposome composition prepared in Preparation Example 12 according to the present invention.

### Mode for Carrying Out the Invention

### <Phospholipids>

The phospholipid, which is a main lipid constituting a lipid bilayer (hereinafter, sometimes referred also to simply as lipid membrane or liposome membrane) of a liposome composition according to the present invention, is a main component of biomembrane. In general, the phospholipid is an amphipathic substance which has both a hydrophobic group composed of a long chain alkyl group and a hydrophilic group composed of a phosphate group in its molecule. Preferred examples of the phospholipid include: glycerophosphoric acids such as phosphatidylcholine (= lecithin), phosphatidylglycerol, phosphatidic acid, phosphatidylethanolamine, phosphatidylserine, and phosphatidylinositol; sphingophospholipids such as sphingomyelin; natural or synthetic diphosphatidylphospholipids such as cardiolipin, and their derivatives; hydrogenation products of these phospholipids such as hydrogenated soybean phosphatidylcholine (HSPC), hydrogenated egg yolk phosphatidylcholine, distearoylphosphatidylcholine, dipalmitoylphosphatidylcholine, and dimyristoylphosphatidylcholine. The phospholipids can be used either singly or in combination of a plurality of ones of them.

### <Other Additives than Phospholipids>

The liposome composition according to the present invention includes, together with the above-mentioned main component, cholesterol, and may include other membrane component(s). For example, the liposome composition can contain membrane stabilizers, antioxidants and the like, as required. Examples of the antioxidants include ascorbic acid, uric acid, and tocopherol homologues, or vitamin E. While tocopherol include four isomers, namely, α-, β-, γ- and δ-tocopherols, any of them can be used in the present invention.

Incidentally, the composition of the lipid bilayer of the liposome composition according to the present invention is preferably less than 100 to 50 mol% of phospholipid and up to to 50 mol% of cholesterol, more preferably 70 to 50 mol% of phospholipid and 30 to 50 mol% of cholesterol.

The liposome composition of the present invention includes a first liposome having an outer membrane comprised of a multilayered lipid bilayer, and a plurality of second liposomes which are accommodated in a first liposome inner region defined by the outer membrane and each of which has an outer membrane comprised of a multilayered lipid bilayer. The liposome composition has second liposome inner regions each defined by the outer membrane of each of the second liposomes.

The liposome composition according to the present invention includes, as modes thereof, an empty liposome in which no drug is encapsulated, and a liposome in which a drug is encapsulated.

The outside diameter of the first liposome is preferably 1 to 20 µm, more preferably 3 to 10 µm, from a viewpoint that such an outside diameter promises excellent sustained release properties and enables easy administration even through thin needles. In addition, the outside diameter of the second liposomes is not particularly limited; preferably, however, the outside diameter of the second liposomes is 100 to 800 nm, from a viewpoint of drug encapsulation amount and excellent sustained release properties.

In the present invention, the plurality of second liposomes are present independently from each other in the first liposome, and the number of them is not particularly limited.

The liposome composition of the present invention has an ion gradient formed at least between each of the second liposome inner regions and the outside of the first liposome. Hereinafter, the term "ion" as simply used means an ion forming the ion gradient. In the present invention, that an ion gradient is formed between each of the second liposome inner regions and the outside of the first liposome means any of: (1) that a difference in ion concentration is present across the outer membrane of a second liposome, between the second liposome inner region and both of the first liposome inner region and the outside of the first liposome; (2) that a difference in ion concentration is present across the outer membrane of the first liposome, between the second liposome inner region as well as the first liposome inner region and the outside of the first liposome; and (3) that a difference in ion concentration is present across the outer membrane of the second liposome, between the second liposome inner region and the first liposome inner region and that a difference in ion concentration is present across the outer membrane of the first liposome, between the first liposome inner region and the outside of the first liposome (in this case, the ion concentration in the first liposome inner region is a value between the ion concentration in the second liposome inner region and the ion concentration in the outside of the first liposome).

In the present invention, it is preferable, from a viewpoint of introduction of much drug, that the ion concentration in the second liposome inner regions is the highest. In addition, a setting can be made in which (the ion concentration in the second liposome inner regions) ≥ (the ion concentration in the first liposome inner region) > (the ion concentration in the outside of the first liposome). Besides, a setting can be made in which (the ion concentration in the second liposome inner regions) > (the ion concentration in the first liposome inner region) ≥ (the ion concentration in the outside of the first liposome). In addition, a setting can be made in which (the ion concentration in the second liposome inner regions) > (the ion concentration in the first liposome inner region) > (the ion concentration in the outside of the first liposome). The case where (the ion concentration in the second liposome inner regions) = (the ion concentration in the first liposome inner region) = (the ion concentration in the outside of the first liposome) is excluded from the present invention. Where proton gradient (pH gradient) is used as the ion gradient, a high ion concentration (proton concentration) corresponds to a low pH. In other words, it is preferable, in this case, that the pH in the second liposome inner regions is the lowest.

Before and after the introduction of a drug into empty liposomes, the shape and outside diameter of the first liposome as well as the shape and outside diameter of the second liposomes are substantially the same. In the liposomes into which a drug has been introduced, the outside diameter of the first liposome and the outside diameter of the second liposomes are the same as those in the empty liposomes into which no drug has been introduced.

In the case where a drug is enveloped in the liposome composition according to the present invention, such a liposome composition can contain the drug in the second liposome inner regions or in the second liposome and first liposome inner regions.

The amount of the drug contained in the liposome composition is not particularly limited, and can be appropriately controlled according to the use of the composition. The amount of the drug, in terms of molar ratio [drug (mol)/total lipid (mol)] thereof based on the total lipid possessed by the liposome composition (the total amount of lipid(s) used in preparation of the liposome composition), is preferably not less than 0.05, and may be 0.06 to 0.14.

### <Ion Gradient Method>

The ion gradient method is a method in which an ion gradient is formed between the inside and the outside of a liposome membrane, and a drug added to the outside is transmitted through the liposome membrane according to the ion gradient, whereby the drug is encapsulated in the inside of the liposome. The ion gradient is preferably proton gradient (pH gradient). In the ion gradient method, empty liposomes in which no drug is encapsulated are prepared, and a drug is added to an outer liquid around the empty liposomes, whereby the drug can be introduced into the liposomes.

The present invention provides a liposome composition in which to encapsulate a drug by the ion gradient method, and a liposome composition in which a drug has been encapsulated by the ion gradient method. Among others, a pH gradient method in which pH gradient is used as the ion gradient is most favorably applied.

As a method of forming a pH gradient, a liposome is formed by using an acidic-pH buffer (for example, a citric acid solution of pH 2 to 3) as a first inner aqueous phase and/or a second inner aqueous phase, and then the pH in the outside of the first liposome is controlled to within the vicinity of neutrality (for example, a buffer of pH 6.5 to 7.5), whereby a mode can be realized in which a pH gradient is formed such that the inside of the second liposomes and the inside of the first liposome are at lower pH whereas the outside of the first liposome is at a higher pH.

In addition, a pH gradient can also be formed through an ammonium ion gradient. In this case, for example, a liposome is formed by using an ammonium sulfate solution as a first inner aqueous phase and/or a second inner aqueous phase, and then the ammonium sulfate in the outer aqueous phase for the first liposome is removed or diluted, whereby an ammonium ion gradient is formed at least between the inside of the second liposomes as well as the first liposome and the outside of the first liposome. This ensures that due to the ammonium ion gradient thus formed, outflow of ammonia from the inner aqueous phases in the first liposome and the second liposomes into the outer aqueous phase for the first liposome takes place. As a result, protons left by the ammonia are accumulated in the inner aqueous phases, whereby a pH gradient is formed, and the inner aqueous phases in the first liposome and the second liposomes become more acidic than the outer aqueous phase for the first liposome.

### <Drug to be Encapsulated>

As the drug to be encapsulated in the liposome composition of the present invention, a drug can be used, without any special restriction, insofar as it can be encapsulated into liposomes by the ion gradient method. Such a drug is preferably an ionizable amphipathic drug, more preferably an amphipathic weakly basic drug. In addition, from a viewpoint of effect, the drug is preferably a drug for which sustained release properties in local administration are expected, particularly any of drugs for treatment of cerebral vascular disorder, Parkinson's disease, dementia, etc., analgesic agents, local anesthetics, and anti-malignancy agents. Examples of these drugs include donepezil, rivastigmine, galanthamine, physostigmine, heptylphysostigmine, phenserine, tolserine, symserine, thiatolserine, thiacymserine, neostigmine, huperzine, tacrine, metrifonate, minocycline, fasudil hydrochloride, nimodine, morphine, bupivacaine, ropivacaine, levobupivacaine, tramadol, lidocaine, and doxorubicin. Other examples include dopamine, L-DOPA, serotonin, epinephrine, codeine, meperidine, methadone, morphine, atropine, decyclomine, metixene, propantheline, imipramine, amitriptyline, doxepin, desipramine, quinidine, propranolol, chlorpromazine, promethazine, and perphenazine.

### <Liposome First Inner Aqueous Phase Solution>

In the process of producing the liposome composition according to the present invention, a first inner aqueous phase solution to be used in a step of preparing a first emulsion contains a compound for forming the ion gradient.

The ion for forming the ion gradient is preferably the proton, as above-mentioned. In addition, examples of the compound for forming the ion gradient (pH gradient) include those compounds which generate proton, ammonium ion or a protonated amino group through ionization. Specific examples of such a compound include: sulfates such as ammonium sulfate, dextran sulfate, and chondroitin sulfate; hydroxides; phosphoric acid, glucuronic acid, citric acid, carbonic acid, hydrogencarbonates, nitric acid, cyanic acid, acetic acid, benzoic acid, and their salts; halides such as bromides, and chlorides; inorganic or organic anions; and anionic polymers.

In the case where a weakly basic drug (for example, any of the above-mentioned ones) is encapsulated in the inner aqueous phase (at least the second inner aqueous phase) in the liposome composition according to the present invention by the pH gradient method, the drug is protonated by the protons present in the inner aqueous phase, to be thereby electrically charged. As a result, the drug is hampered from diffusing to the outside of the liposome, so that the drug is maintained in the liposome inner aqueous phase.

Besides, in the case where the compound for forming the ion gradient is ionized, anions such as sulfate ions are generated together with the ions (cations) for forming the ion gradient such as protons. In this case, if the anion forms a salt or complex with the protonated weakly basic drug, the drug can be maintained in the inner aqueous phase more stably. In other words, the compound for forming the ion gradient can be a compound which generates, through ionization, a counter ion (anion) for the basic drug and which is capable of forming a salt or complex with the basic drug. Such a counter ion is not specifically restricted so long as it is a pharmaceutically permissible anion; preferably, however, the counter ion is sulfate ion. As a compound for generating the sulfate ion, ammonium sulfate is generally used, but the compound may also be selected from other compounds such as dextran sulfate and chondroitin sulfate. In addition, other examples of the counter ion include anions generated through ionization from hydroxides, phosphates, glucuronates, citrates, carbonates, hydrogencarbonates, nitrates, cyanates, acetates, benzoates, bromides, chlorides, and other inorganic or organic anions, or anionic polymers, etc.

In the present invention, the concentration of the compound for forming the ion gradient in the first inner aqueous phase solution is preferably 50 to 500 mM, more preferably 100 to 300 mM.

In the method of producing the liposome composition according to the present invention, the solvent to be used in preparation of the lipid-containing solution in the step of preparing the first emulsion is a water-miscible solvent. The water-miscible solvent means a solvent which dissolves the phospholipid(s) and cholesterol and other membrane component(s) used in the production of the liposome composition according to the present invention and which is miscible with water. Examples of the water-miscible solvent include ethanol, methanol, isopropyl alcohol, and butanol.

Solvents which are not miscible with water (referred to also as water-immiscible solvents; examples include water-immiscible organic solvents such as chloroform) are not used in the present invention. When a water-immiscible solvent is used in the step of preparing the first emulsion, the liposome obtained does not have a form in which a plurality of small liposomes and a first inner aqueous phase are contained in a large liposome; instead, the liposome obtained merely has a form [so-called multivesicular liposome (MVL)] in which individual liposomes are gathering simply, like expanded polystyrene.

The amount of lipid(s) as a liposome raw material (the total amount of phospholipid(s) and other lipid(s)) is preferably 20 to 100 mass%, more preferably 20 to 60 mass%, based on the water-miscible solvent.

In the first emulsion (the mixture of the lipid-containing water-miscible solvent and the ion-containing first inner aqueous phase solution), other component(s) than the components capable of constituting the lipid bilayer can fill up the inner regions of the second liposomes constituting the liposome composition of the present invention. Part of a second inner aqueous phase solution, which will be described later, may be additionally mixed in the inner regions of the second liposomes.

The method for preparing the first emulsion is not specifically restricted, and conventionally known methods can be used.

In the case where the pH gradient method is used, the pH of the inner aqueous phase (the first and/or second liposome inner region) can be controlled, as required. For example, in the case where citric acid as a compound for forming an ion gradient is used in the first inner aqueous phase solution, it is necessary to preliminarily form a pH gradient between the inner aqueous phase (the second liposome inner regions) and the outer aqueous phase (the first liposome inner region and/or the outside of the first liposome); in this case, the difference in pH between the inner aqueous phase and the outer aqueous phase is preferably not less than three.

Besides, in the case where ammonium sulfate is used, a pH gradient is formed by chemical equilibrium, which makes it unnecessary to preliminarily control the pH of the inner aqueous phase solution. In this case, if the same solution as the outer aqueous phase is used as the second inner aqueous phase, formation of an ion gradient begins from the time of formation of the second emulsion, and a further gradient is formed by replacement of the outer liquid. In the case where the same ammonium sulfate solution as the first inner aqueous phase is used as the second inner aqueous phase, it is considered that an ion gradient is formed at the time of replacement of the outer liquid.

In the preparation of the liposome composition according to the present invention, the lipid-containing water-miscible solvent and the first inner aqueous phase solution to be added thereto can be used in a volume ratio (of the first inner aqueous phase solution to the water-miscible solvent) in a range from 0.7 to 2.5, preferably from 1.0 to 2.0.

### <Liposome Second Inner Aqueous Phase Solution>

In the present invention, after the preparation of the first emulsion by adding the first inner aqueous phase solution to the lipid-containing water-miscible solvent, a step of adding the second inner aqueous phase solution to the first emulsion is conducted, in which the second inner aqueous phase solution is not specifically restricted. Examples of the second inner aqueous phase solution include the same solution as the first inner aqueous phase, a HEPES solution, a NaCl solution, and aqueous solutions of sugar such as glucose and sucrose, among which preferred is the same solution as the first inner aqueous phase. In addition, it is most preferable that the first inner aqueous phase and the second inner aqueous phase are each an aqueous ammonium sulfate solution. The first emulsion and the second inner aqueous phase solution to be added thereto are used in a volume ratio [of the second inner aqueous phase solution to the first emulsion (= first inner aqueous phase solution + water-miscible solvent)] of from 0.7 to 2.5, more preferably in a range of from 1.0 to 1.5.

In the second emulsion, other component(s) than the component(s) capable of constituting the lipid bilayer can fill up the first liposome inner region (exclusive of the second liposomes) constituting the liposome composition of the present invention. The first liposome inner region (exclusive of the second liposomes) may contain part of the first emulsion.

The method for preparation of the second emulsion is not specifically restricted, and conventionally known methods can be used.

### <Liposome Outer Aqueous Phase Solution>

The process for producing the liposome composition according to the present invention includes a step of replacing the outer aqueous phase of the second emulsion with an aqueous solution which is lower than the first inner aqueous phase solution in the concentration of the compound for forming the ion gradient.

Where the outer aqueous phase of the first liposome after preparation of the second emulsion is changed by replacement of the liposome second inner aqueous phase solution or the mixed liquid containing the liposome first inner aqueous phase solution and the liposome second inner aqueous phase solution with an aqueous solution which is at least lower than the first inner aqueous phase solution in the concentration of the compound for forming the ion gradient, it is ensured that an ion gradient is formed at least between each of the second liposome inner regions and the outside of the first liposome, that the water-miscible solvent is removed from within the liposome composition system, and that the liposome obtained can be provided with the form possessed by the liposome composition according to the present invention.

As the outer aqueous phase for replacement that is used in the process for production of the liposome composition according to the present invention, an aqueous solution at least lower than the first inner aqueous phase solution in the concentration of the compound for forming the ion gradient is used. Specifically, a HEPES solution, a NaCl solution, or an aqueous solution of sugar such as glucose and sucrose is used. The pH of the outer aqueous phase is desirably adjusted by use of a buffer. Taking into account the decomposition of lipid and the pH gap at the time of administration into a living body, the pH is preferably controlled to within a range of pH 5.5 to 8.5, more preferably a range of pH 6.5 to 7.5. Osmotic pressures of the inner aqueous phase and the outer aqueous phase for the liposome are not particularly limited insofar as the osmotic pressures are controlled to within such ranges that the liposome would not be broken by the difference between the osmotic pressures. In consideration of physical stability of the liposome, however, a slighter difference in osmotic pressure is more desirable.

One preferred embodiment of the outer aqueous phase for replacement is an aqueous solution that is lower than the first inner aqueous phase solution and the second inner aqueous phase solution in the concentration of the compound for forming the ion gradient.

The process for producing the liposome composition according to the present invention may further include a step of introducing a drug into the inside of the liposome composition by a driving force due to the ion gradient. In the step of introducing a drug into the inside of the liposome composition by the driving force due to the ion gradient, for example, the drug is dissolved in water or the like. The resulting drug solution is added to a liposome mixture obtained upon replacement of the liposome outer aqueous phase with the liposome outer aqueous phase solution, followed by blending the admixture. The blended admixture is stirred with heating to or above a phase transition temperature of the liposome membrane, whereby a liposome in which the drug is encapsulated can be produced.

### <Administering Method>

The method for administering the liposome composition according to the present invention is not specifically restricted; preferably, however, the liposome composition is administered non-perorally and locally. For instance, subcutaneous, intramuscular, intraperitoneal, intrathecal, extradural or intraventricular administration can be selected. The administering method can be appropriately selected according to the relevant symptom. As a specific method for administration, the liposome composition can be administered by use of a syringe or a spray-type device. In addition, the administration can be carried out through a catheter inserted in a living body, for example, in a body lumen, for instance, in a blood vessel.

### Examples

Now, the present invention will be described more in detail below by showing Examples, but the invention is not to be restricted to the Examples.

The concentration and particle diameter of each of drug-filled liposomes prepared in Examples were determined as follows.

Phospholipid Concentration (mg/mL): Phospholipid concentration in a liposome suspension that is quantified by high performance liquid chromatography or phospholipids determination.

Cholesterol Concentration (mg/mL): Cholesterol concentration in a liposome suspension that is quantified by high performance liquid chromatography.

Total Lipid Concentration (mol/L): Total mol concentration (mM) of lipid(s) as membrane component(s) that is calculated from the phospholipid concentration and the cholesterol concentration.

Drug Concentration (mg/mL): The liposome composition was diluted with RO water (reverse osmosis-purified water) so that the total lipid concentration of the preparation obtained above would be about 20 to 30 mg/mL. Then, the diluted liposome composition was further diluted with methanol by a factor of 20, and the liposome was disintegrated. For the resulting solution, absorbance at 315 nm was quantified by high performance liquid chromatography using a UV absorptiometer. The concentration of encapsulated donepezil hydrochloride is shown in drug amount (mg)/total preparation amount (mL).

Drug Support Amount (molar ratio of drug/total lipid): The concentration of donepezil hydrochloride encapsulated in the liposomes is shown in molar ratio of drug/total lipid, calculated from the ratio of the drug concentration to the total lipid concentration.

Concentration of Donepezil Hydrochloride in Plasma (mg/mL): Sampled plasma was treated, and, for a supernatant obtained finally by centrifugation, fluorescence at an excitation wavelength (Ex) of 322 nm and a detection wavelength (Em) of 385 nm was quantified by high performance liquid chromatography using a fluorophotometer.

Particle Diameter (µm): Average particle diameter of the first liposome measured by a light scattering diffraction particle size distribution analyzer Beckman Coulter LS230.

The abbreviations and molecular weights of components used are set forth below.
HSPC: Hydrogenated soybean phosphatidylcholine (molecular weight 790, SPC3 produced by Lipoid GmbH)
SPC: Soybean Phosphatidylcholine (molecular weight 779, NOF Corporation)
DMPC: Dimyristoylphosphatidylcholine (molecular weight 677.9, NOF Corporation)
Chol: Cholesterol (molecular weight 388.66, produced by Solvay S.A.)
PEG5000-DSPE: Polyethylene glycol (molecular weight 5,000)-Phosphatidylethanolamine (molecular weight 6081, NOF Corporation)
Donepezil hydrochloride (molecular weight 415.95, UINAN CHENGHUI-SHUANFDA Chemical Co., Ltd.)

### <Preparation of Different Inner Aqueous Phases>

### (Preparation Examples 1 to 4)

### (1) Preparation of empty liposome

HSPC and cholesterol in respective amounts of 1.41 g and 0.59 g were weighed so that HSPC/Chol = 54/46 (molar ratio, here and hereafter), then 4 mL of anhydrous ethanol was added thereto, and dissolution was effected by heating. To the ethanol solution of lipid thus obtained by dissolution, there was added 100 mM (Preparation Example 1), 150 mM (Preparation Example 2) or 250 mM (Preparation Example 3) of an aqueous ammonium sulfate solution or 300 mM of an aqueous citric acid solution (pH 3.0) (Preparation Example 4) heated to about 70°C in the same amount (4 mL) as ethanol. Each admixture was heated and stirred for about ten minutes, to form an emulsion. Furthermore, the emulsion was admixed with 10 mL of 20 mM HEPES/0.9% sodium chloride (pH 7.5) heated to about 70°C, followed by heating and stirring for about ten minutes. After the heating was over, the liposomes were immediately cooled with ice.

### (2) Formation of pH gradient

The liposomes obtained as above were dispersed in 20 mM HEPES/0.9% sodium chloride (pH 7.5) added thereto, followed by centrifugation at 3,500 rpm for 15 minutes, to precipitate the liposomes. Thereafter, the supernatant was removed, and subsequently the liposomes were dispersed in 20 mM HEPES/0.9% sodium chloride of pH 7.5 added thereto, followed by centrifugation in the same manner as above. This step was repeated three times, followed by re-dispersing in 20 mM HEPES/0.9% sodium chloride of pH 7.5, to form a pH gradient.

### (3) Introduction of drug by pH gradient

After the formation of the ion gradient, the amounts of HSPC and cholesterol of the liposomes were determined, and total lipid concentration was calculated. Based on the total lipid concentration thus calculated, the amount of donepezil hydrochloride (DNP, molecular weight 415.95) for realizing a DNP/total lipid (mol/mol) ratio of 0.16 was calculated. After the required amount of DNP was weighed, a DNP solution (drug solution) of a concentration of 20 mg/mL was prepared by use of RO water. A predetermined amount of DNP solution preliminarily heated to 65°C was added to the liposome solution heated to 65°C, followed by heating and stirring at 65°C for 60 minutes, to effect introduction of the drug. After the introduction of the drug, the liposomes were immediately cooled with ice.

### (4) Removal of unencapsulated drug

After the introduction of the drug, the liposomes were dispersed in 20 mM HEPES/0.9% sodium chloride (pH 7.5) added thereto, followed by centrifugation at 3,500 rpm for 15 minutes, to precipitate the liposomes. Thereafter, the supernatant was removed, and subsequently the liposomes were dispersed in 20 mM HEPES/0.9% sodium chloride (pH 7.5) added thereto, followed by centrifugation in the same manner as above. This step was repeated three times, thereby removing the unencapsulated drug.

For the liposome compositions of Preparation Examples 1 to 4 obtained by the producing method according to the present invention as above-mentioned, the first inner aqueous phases, membrane compositional ratios, drug support amounts (molar ratios of drug/total lipid) and particle diameters are set forth in Table 1. As a result of electron microscope observation, the liposome compositions according to the present invention appeared as shown in FIG. 1, in which a multiplicity of vesicles (second liposomes) are present in each liposome (first liposome), and in which the outer membrane of each liposome is composed of a multilayered lipid bilayer (multilayered lipid bilayers, or a multiple of lipid bilayers). Moreover, notwithstanding that the liposome has the thick multilayered lipid bilayer and contains therein the multiplicity of vesicles each having the multilayered lipid bilayer in the same manner, a pH gradient sufficient for introduction of a drug can be formed between the inside and the outside of the liposomes after the formation of the liposomes. Consequently, the drug can be encapsulated highly efficiently, attendant on the pH gradient.

FIG. 1 is a photograph upon transmission electron microscope (TEM) observation of a section of the liposome after the drug introduction, produced in Preparation Example 2 according to this Example of the present invention. The magnification is 32,000. The liposome shown in FIG. 1 is divided substantially at the center of the liposome. The liposome shown in FIG. 1 includes a first liposome having an outer membrane composed of a multilayered lipid bilayer (multilayered lipid bilayers, or a multiple of lipid bilayers), and a plurality of second liposomes which are accommodated in the first liposome inner region defined by the outer membrane and each of which has an outer membrane composed of a multilayered lipid bilayer (multilayered lipid bilayers, or a multiple of lipid bilayers). In FIG. 1, the outside diameter of the first liposome is about 4 µm, and the outside diameter of the second liposomes is 100 to 800 nm.

In addition, for the liposome compositions of Preparation Examples 1 to 4 obtained by the producing method according to the present invention as above-mentioned, it has been made clear that the encapsulation amount of the drug is enhanced depending on an increase in the concentration of ammonium sulfate in the inner aqueous phase. This is considered to be because a drug holding capability was enhanced attendant on the amount of protons remaining in the inner aqueous phase. It is considered, therefore, that an ammonium sulfate concentration of not less than 150 mM is preferred, in order to obtain a higher drug encapsulation amount. Besides, also in the case where a citric acid solution of pH 3.0 was used as the inner aqueous phase in place of ammonium sulfate, a liposome having a high drug encapsulation amount was obtained in the same manner.

**[Table 1]**

| | First inner aqueous phase | Lipid composition (mol/mol) | Volume ratio, I | Volume ratio, II | Drug | Drug/Total lipid (mol/mol) | Average particle diameter of first liposome (µm) |
|---|---|---|---|---|---|---|---|
| Preparation Example 1 | 100mM AS | HSPC/Chol=54/46 | 1 | 1.25 | Donepezil hydrochloride | 0.06 | 3.5 |
| Preparation Example 2 | 150 mM AS | HSPC/Chol=54/46 | 1 | 1.25 | Donepezil hydrochloride | 0.09 | 6.8 |
| Preparation Example 3 | 250 mM AS | HSPC/Chol=54/46 | 1 | 1.25 | Donepezil hydrochloride | 0.11 | 7.9 |
| Preparation Example 4 | pH3.0 300 mM CA | HSPC/Chol=54/46 | 1 | 1.25 | Donepezil hydrochloride | 0.14 | 6.0 |
| Preparation Example 5 | 150 mM AS | HSPC/Chol=54/46 | 2 | 1.6 | Donepezil hydrochloride | 0.11 | 6.4 |
| Preparation Example 6 | 150 mM AS | DMPC/Chol=54/46 | 1 | 1.25 | Donepezil hydrochloride | 0.11 | 4.7 |
| Preparation Example 7 | 150 mM AS | HSPC/Chol=54/46 | 1 | 1 | Donepezil hydrochloride | 0.07 | 6.7 |
| Preparation Example 8 | 150 mM AS | HSPC/Chol=54/46 | 1 | 1.6 | Donepezil hydrochloride | 0.09 | 6.8 |
| Preparation Example 9 | 150 mM AS | HSPC/Chol=54/46 | 1 | 2 | Donepezil hydrochloride | 0.09 | 5.5 |
| Preparation Example 10 | 150 mM AS | HSPC/Chol=54/46 | 2 | 1 | Donepezil hydrochloride | 0.09 | 7.8 |
| Preparation Example 11 | 150 mM AS | HSPC/Chol=54/46 | 2 | 2 | Donepezil hydrochloride | 0.09 | 7.3 |
| Preparation Example 12 | 150 mM AS | HSPC/Chol=54/46 | 1 | 1.6 | Bupivacaine hydrochloride, | 0.08 | 8.0 |
| Preparation Example 13 | 250 mM AS | HSPC/Chol=54/46 | 1 | 1.6 | Bupivacaine hydrochloride | 0.11 | 8.1 |
| Preparation Example 14 | 150 mM AS | HSPC/Chol=54/46 | 1 | 1.25 | Ropivacaine hydrochloride | 0.09 | 9.3 |
| Preparation Example 15 | 250 mM AS | HSPC/Chol=54/46 | 1 | 1.25 | Tramadol hydrochloride | 0.09 | 9.1 |
| Comparative Example 5 | 150 mM AS | HSPC/Chol=54/46 | 0.5 | 1.6 | Donepezil hydrochloride | 0.02 | 13.4 |
| Comparative Example 6 | 150 mM AS | HSPC/Chol=54/46 | 9.0 | 0 | Donepezil hydrochloride | 0.07 | 8.1 |
| Comparative Example 7 | 150 mM AS | HSPC/Chol=54/46 | 9.0 | 1.6 | Donepezil hydrochloride | 0.05 | 9.6 |

| | | | | | | | |
|---|---|---|---|---|---|---|---|
| Volume ratio, I: Volume ratio of first inner aqueous phase solution/ethanol Volume ratio, II: Volume ratio of second inner aqueous phase/(first inner aqueous phase + ethanol) AS: ammonium sulfate CA: citric acid | | | | | | | |

### <Investigation of Different Inner Aqueous Phase Volumes>

### (Preparation Example 5)

HSPC and cholesterol in respective amounts of 1.41 g and 0.59 g were weight so that HSPC/Chol = 54/46, and they were dissolved in 4 mL of an ethanol solution. After the dissolution, the ethanol solution of lipid was admixed with two-fold amount (8 mL) of a 150 mM aqueous ammonium sulfate solution, followed by heating and stirring for about ten minutes. Subsequently, 19 mL of a 150 mM aqueous ammonium sulfate solution was added thereto, and the resulting admixture was heated and stirred for about ten minutes. Thereafter, a pH gradient was formed and drug introduction and removal of the unencapsulated drug were conducted, in the same manner as in Preparation Examples 1 to 4. As a result, a high drug encapsulation amount was obtained in the same manner as in Preparation Examples 1 to 4, as shown in Table 1.

### (Preparation Example 6)

As a phospholipid, DMPC having a small alkyl group chain length was used. The DMPC and cholesterol in respective amounts of 2.70 g and 1.30 g were weighed so that DMPC/Chol = 54/46, and were dissolved in 4 mL of an ethanol solution. Subsequently, 4 mL of a 150 mM aqueous ammonium sulfate solution was added to the ethanol solution of DMPC and cholesterol, followed by heating and stirring for about ten minutes. Thereafter, 10 mL of a 150 mM aqueous ammonium sulfate solution was added thereto, followed by heating and stirring for about ten minutes. Subsequently, a pH gradient was formed and drug introduction and removal of the unencapsulated drug were conducted, in the same manner as in Preparation Examples 1 to 4.

As a result, it was found out that, also in this case where the alkyl group chain length of the phospholipid was small, a high drug encapsulation amount was obtained in the same manner as in Preparation Examples 1 to 4, as shown in Table 1.

### <Preparation of DNP Liposome by Other Method (Passive Method) than Ion Gradient Method>

### (Comparative Example 1)

In drug introduction, the passive method (which is a conventional method) was used in place of the ion gradient method. The passive method is a method in which liposomes are prepared by preliminarily dissolving a drug in an inner aqueous phase. A predetermined amount of donepezil hydrochloride was preliminarily dissolved in physiological saline used as the first inner aqueous phase solution. Thereafter, liposome preparation was conducted in the same manner as in Preparation Examples 1 to 4. Incidentally, physiological saline was used also as the outer aqueous phase.

As a result, it was made clear that encapsulation efficiency and the drug encapsulation amount are conspicuously lowered, as compared with the liposomes obtained by the method according to the present invention, as shown in Table 2. Furthermore, the liposome compositions according to Comparative Example 1 and the present invention were compared with each other as to drug release properties by use of an in-vitro evaluation system. As a result, it was made clear that the drug release was much faster in Comparative Example 1 than in the present invention.

From the foregoing, it was found clearly that in order to secure a drug encapsulation amount at a clinically sufficient level and to obtain long-term sustained release properties, it is important to introduce a drug by the ion gradient method, particularly the pH gradient method. It was also made clear that where a drug is introduced by the ion gradient method, particularly the pH gradient method, the drug is protonated in the inside of the liposomes, and the liposomes have a layered structure as shown in FIG. 1, whereby longer-term sustained release properties can be obtained.

**[Table 2]**

| Comparative Example | First inner aqueous phase solution | Lipid composition (mol/mol) | Drug support amount | Particle diameter (µm) |
|---|---|---|---|---|
| | | | Drug/Total lipid (mol/mol) | |
| 1 | Physiological saline/ Donepezil solution | HSPC/Chol = 54/46 | 0.01 | 3.8 |

### <Preparation of Donepezil Liposome by Different Producing Methods>

### (Comparative Example 2) Extrusion Method-1 (particle diameter: around 300 nm)

HSPC and cholesterol in respective amounts of 0.71 g and 0.29 g were weighed so that HSPC/Chol = 54/46, and were dissolved with heating in 1 mL of anhydrous ethanol added thereto. The ethanol solution of lipid thus obtained, in an amount of 1 mL, was admixed with 9 mL of a 250 mM aqueous ammonium sulfate solution (inner aqueous phase) heated to about 70°C, followed by stirring by a ultrasonic device with heating, to prepare a crude liposome suspension. The crude liposome suspension thus obtained was passed sequentially through a filter (pore diameter 0.4 µm, Whatman plc; five times) attached to an extruder (The Extruder T.10, Lipexbiomembranes Inc.) heated to about 70°C, to prepare empty liposomes sized around 300 nm. Subsequently, while maintaining the liposomes in a heated state, an aqueous PEG5000-DSPE solution (37.7 mg/mL) was immediately added in such an amount as to be 0.75 mol% based on the total lipid, followed by heating and stirring, whereby membrane surfaces (outer surfaces) of the liposomes were modified with PEG. After the heating was over, the liposomes were immediately cooled with ice. The PED-modified liposomes thus ice-cooled were subjected to outer liquid replacement by use of gel filtration replaced sufficiently with an outer aqueous phase solution (20 mM HEPES/0.9% sodium chloride solution (pH 7.5)). Thereafter, drug introduction was conducted so that drug/total lipid (mol/mol) = 0.16. Subsequently, removal of the unencapsulated drug was conducted by use of gel filtration replaced sufficiently with 20 mM HEPES/0.9% sodium chloride solution (pH 7.5).

### (Comparative Example 3) Extrusion Method-2 (particle diameter: around 1 to 2 µm)

Preparation was conducted by use of an extruder in the same manner as in Comparative Example 2, except that a filter with a pore diameter of 2 µm was attached to the extruder, and the crude liposome suspension was passed through the filter five times, to obtain empty liposomes. The preparation was conducted by carrying out the drug introduction and removal of the unencapsulated drug in the same manner as in Comparative Example 2, to obtain multilamellar liposomes sized around 1 to 2 µm.

### (Comparative Example 4) Lipid Membrane Introduction Method

An aqueous citric acid hydrochloric acid solution of pH 6.5 as the first inner aqueous phase solution was added to an ethanol solution containing HSPC and donepezil dissolved therein, whereby donepezil hydrochloride was encapsulated in the lipid membrane. Donepezil liposomes were obtained in the same manner as in Comparative Example 1, except for the just-mentioned points.

For the donepezil liposomes obtained in Comparative Examples 2 to 4, the first inner aqueous phases, membrane compositional ratios, drug support amounts (molar ratios of drug/total lipid) and particle diameters are set forth in Table 3.

As a result, for the liposome compositions (Comparative Examples 2 and 3) with small particle diameters prepared by the extrusion method, high drug encapsulation amounts were obtained. On the other hand, for the preparation (Comparative Example 4) in which the drug was encapsulated in the lipid membrane, the drug/total lipid ratio was comparatively low, and the encapsulation efficiency was about 33%.

**[Table 3]**

| Comparative Example No. | First inner aqueous phase solution | Lipid composition (mol/mol) | Drug support amount | Particle diameter (µm) |
|---|---|---|---|---|
| | | | Drug/Total lipid (mol/mol) | |
| 2 | 250 mM ammonium sulfate | HSPC/Chol = 54/46 | 0.13 | 0.29 |
| 3 | 150 mM ammonium sulfate | | 0.15 | 1.7 |
| 4 | pH 6.5 citric acid | HSPC = 100 | 0.05 | 7.4 |

### <Donepezil Liposome Drug Dynamics 1>

The donepezil liposome compositions prepared in Preparation Examples 2, 3 and 4 and Comparative Examples 2, 3 and 4 as well as donepezil used alone were subjected to a drug dynamics test. The donepezil liposome compositions in the volumes set forth in Table 4 as donepezil hydrochloride amount were each administered subcutaneously into a back part of a rat. Incidentally, for donepezil hydrochloride used alone, intravenous administration was conducted as well as the subcutaneous administration. After 1, 4, 8, 24, 48, 72, 96, 168, 192, 216, 240, 264, and 336 hours from the administration, blood was sampled from a tail vein, and subjected to centrifugation (6,000 rpm, ten minutes, at 4°C), whereby plasma was obtained fractionally. The thus obtained plasma was treated, and the fluorescence intensity at an excitation wavelength (Ex) of 322 nm and a detection wavelength (Em) of 385 nm was determined by high performance liquid chromatography, thereby determining the concentration of donepezil hydrochloride in each plasma. The results are shown in FIGS. 2 and 3.

**[Table 4]**

| Preparation | Dose | Results of pharmacokinetic profile |
|---|---|---|
| Donepezil alone, intravenous administration | IV 2.5 mg/kg | FIG. 2 |
| Donepezil alone, subcutaneous administration | SC 2.5 mg/kg | |
| Comparative Example 2 | SC 2.5 mg/kg | |
| | SC 5 mg/kg | |
| Comparative Example 4 | SC 15 mg/kg | FIG. 3 |
| Preparation Example 2 | SC 25 mg/kg | |
| Preparation Example 3 | | |
| Preparation Example 4 | | |
| Comparative Example 3 | | |

As shown in FIG. 2, the concentration of donepezil hydrochloride in blood when donepezil hydrochloride used alone was administered intravenously or subcutaneously decreased rapidly after the administration, and the detection thereof continued only for eight hours and 48 hours after the administration, respectively. The liposome composition with a particle diameter of around 300 nm prepared in Comparative Example 2 did not show an initial burst, unlike donepezil used alone; besides, although it showed sustained release until 48 hours passed, its concentration already decreased below 10 ng/ml in 48 hours after the administration. The reason is probably as follows. When the particle diameter is comparatively small as about 300 nm, the liposomes are liable to diffuse in the administration region, and donepezil hydrochloride is supposed to be transferred into lymph nodes or into blood together with the liposomes. It is therefore considered that the liposomes are lost early, and the expected sustained release properties cannot be obtained. In addition, in Comparative Example 4 in which the drug was encapsulated in the lipid membrane, the initial release amount is large, and thereafter the concentration of donepezil hydrochloride in blood was lowered rapidly, so that persistent sustained release properties could not be obtained. Probably, due to the high permeability of donepezil hydrochloride through the lipid membrane, the donepezil hydrochloride encapsulated in the membrane was not maintained stably, and, as a result, fast release properties were shown.

On the other hand, as shown in FIG. 3, the liposome compositions obtained in Preparation Examples 2, 3 and 4 of the present invention did not show the initial burst, and showed a marked prolongation of sustained release time; thus, sustained release properties over about two weeks could be obtained. As shown in FIG. 1, the liposome composition according to the present invention contains a multiplicity of vesicles in each liposome, and the liposomes are covered with a thick lipid membrane having a layered structure composed of multiple layers. Due to these structures, permeability of the drug through the lipid membrane is considered to be suppressed. Further, it is considered that since the drug is maintained by the pH gradient method, release is restrained more, with the result that a remarkably long-term sustained release properties could be obtained. Especially, where sulfate ions were present in the inner aqueous phase (Preparation Examples 2 and 3), the sustained release time was prolonged more. Thus, it was suggested preferable to use ammonium sulfate as the inner aqueous phase solution. This is considered to show that an interaction of the protonated drug with the sulfate ions in the inner aqueous phase suppressed the release speed more, and, consequently, the long-term sustained release properties could be achieved.

From the foregoing, it was verified that in order to restrain the initial burst and achieve longer-term sustained release properties, it is important that the liposomes have the form as shown in FIG. 1, the drug is encapsulated by the pH gradient method, and, preferably, sulfate ions are present in the inner aqueous phase.

Besides, as for the liposome composition with a particle diameter of about 1.7 µm prepared by use of the extruder in Comparative Example 3, a high concentration in blood was maintained for four days, after which it was lowered rapidly.

### <Donepezil Liposome Drug Dynamics 2>

With the liposome composition according to the present invention, a high drug encapsulation amount can be obtained, so that the dose of the drug in subcutaneous administration can be enhanced. In view of this, the donepezil liposome compositions prepared in Preparation Examples 5 and 6 were administered subcutaneously into a back part of a rat in a donepezil hydrochloride dose of 50 mg/kg. Furthermore, for comparison, donepezil used alone was administered subcutaneously into a back part of a rat in a dose of 5 mg/kg. For the donepezil used alone, blood was sampled from a tail vein after lapses of 0.5, 1, 5, 10, 30, 120, 480, 1440, and 2880 minutes from the administration. For the liposome compositions, on the other hand, blood was sampled from a tail vein after lapses of 1, 3, 4, 8, 24, 48, 72, 96, 120, 144, 168, 192, 216, 264, 288, 312, and 336 hours from the administration. After the blood sampling, the same treatment as in <Donepezil Liposome Drug Dynamics 1> was conducted, and the concentration of donepezil hydrochloride in each plasma was determined. The results are shown in FIG. 4.

**[Table 5]**

| Preparation | Dose | Results of pharmacokinetic profile |
|---|---|---|
| Donepezil alone | SC 5 mg/kg | |
| Preparation Example 5 | SC 50 mg/kg | FIG. 4 |
| Preparation Example 6 | | |

As shown in FIG. 4, donepezil used alone showed its maximum concentration in blood after 0.5 hour from the administration, followed by a rapid lowering. After 48 hours, the concentration was already below the detection limit.

On the other hand, the liposome compositions according to the present invention prepared in Preparation Examples 5 and 6 did not show any initial burst, and enabled an effective concentration at a clinically sufficient level over 14 days. Especially, in the case of Preparation Example 5, an in-blood concentration of 20 to 30 ng/mL could be kept constantly for 14 days. Furthermore, it showed a profile such as to promise the sustained release properties for more than 14 days. Thus, this liposome composition was verified to be a preparation that is excellent as a sustained release preparation.

### <Investigation of First Inner Aqueous Phase/Ethanol Ratio and Second Inner Aqueous Phase/(First Inner Aqueous Phase + Ethanol) Ratio>

### (Preparation Examples 7 to 11 and Comparative Examples 5 to 7)

HSPC and cholesterol in respective amounts of 1.41 g and 0.59 g were weighed so that HSPC/Chol = 54/46, and were dissolved with heating in 4 mL of anhydrous ethanol added thereto. After the dissolution, the ethanol solution of lipid thus obtained was admixed with a 150 mM aqueous ammonium sulfate solution (first inner aqueous phase) heated to about 70°C, in each of the ratios shown in Table 1, followed by heating and stirring for about ten minutes. Subsequently, a second inner aqueous phase (20 mM HEPES/0.9% sodium chloride buffer (pH 7.5)) was added in each of the ratios shown in Table 1, based on the volume of (the first inner aqueous phase + ethanol), followed further by heating and stirring for about ten minutes. Thereafter, a pH gradient was formed and drug introduction and removal of the unencapsulated drug were carried out in the same manner as in Preparation Examples 1 and 2.

Table 1 shows the first inner aqueous phase/ethanol ratios, the second inner aqueous phase/(first inner aqueous phase + ethanol) ratios, the drug support amounts (molar ratios of drug/total lipid), and the particle diameters, for the liposome compositions prepared in Preparation Examples 7 to 11 and Comparative Examples 5 to 7.

Besides, Table 6 shows the comparison of Preparation Examples 2, 5, and 7 to 11 and Comparative Examples 5 to 7 as to drug support amount.

As a result, it was made clear that Preparation Examples 7 to 11 can yield a drug support amount comparable to those in Preparation Examples 2 and 5, and can yield a comparatively high drug encapsulation amount. In addition, the examples showed substantially the same behavior as to in-vitro release properties.

On the other hand, Comparative Example 5 gave a conspicuously low drug encapsulation amount. The reason is considered to reside in that due to the low first inner aqueous phase/ethanol ratio, the first emulsion was not formed cleanly, and, hence, something like lipid balls (aggregates of lipid) was formed. In addition, in regard of Comparative Examples 6 and 7, it is considered that since the first inner aqueous phase/ethanol ratio is high, something like a large liposome stable at this time point is formed, and the second inner aqueous phase is not liable to influence these structures. Further, as for in-vitro release properties in Comparative Examples 5, 6 and 7, there was a tendency toward a higher initial release speed, as compared with Preparation Examples 2, 5, and 7 to 11. From these results, it is supposed that in Comparative Examples 5, 6 and 7, the inner aqueous phase does not have a clearly formed structure, so that a sufficient amount of drug is not stably encapsulated in the inner aqueous phase, which leads to a slightly lowered drug encapsulation amount and a high initial release rate. On the other hand, in Comparative Example 3, the layers of the lipid membrane are considered to be very thin because of the structure in which a large inner aqueous phase is formed inside, though the liposome has a multilayered membrane. As a result, it is considered that the drug encapsulation amount is very high and the release is also very high.

**[Table 6]**

| Drug/Lipid (mol/mol) | | | | | | |
|---|---|---|---|---|---|---|
| | | Volume ratio of second inner aqueous phase/(first inner aqueous phase + EtOH) | | | | |
| | | 0 | 1/1 | 1.25/1 | 1.6/1 | 2/1 |
| Volume ratio of first inner aqueous phase/EtOH | 0.5/1 | | | | 0.02 (Comparative Example 5) | |
| | 1/1 | | 0.07 (Preparation Example 7) | 0.09 (Preparation Example 2) | 0.09 (Preparation Example 8) | 0.09 (Preparation Example 9) |
| | 2/1 | | 0.09 (Preparation Example 10) | | 0.11 (Preparation Example 5) | 0.09 (Preparation Example 11) |
| | 9/1 | 0.07 (Comparative Example 6) | | | 0.05 (Comparative Example 7) | |

### <Preparation of Bupivacaine Hydrochloride Liposome according to Present Invention>

### (Preparation Examples 12 and 13)

In the same manner as in Preparation Examples 1 to 4, HSPC and cholesterol were weight in respective amounts of 4.23 g and 1.76 g so that HSPC/Chol = 54/46, and were dissolved in 24 mL of an ethanol solution added thereto. After the dissolution, the ethanol solution of lipid was admixed with the same amount (24 mL) of a 150 mM or 250 mM aqueous ammonium sulfate solution, followed by heating with stirring for about ten minutes. Thereafter, 76.8 mL of a 150 mM or 250 mM aqueous ammonium sulfate solution was added, followed by heating with stirring for about ten minutes, and thereafter by immediate cooling with ice. Subsequently, centrifugation was conducted to replace the outer aqueous phase with 10 mM citric acid/0.9% sodium chloride of pH 6.5, thereby forming an ion gradient.

Thereafter, drug introduction was also conducted in the same manner as in Preparation Examples 1 to 4. Bupivacaine hydrochloride was used as the drug. After a required amount of bupivacaine hydrochloride (BPV) was weighed, it was dissolved in RO water to prepare a BVP solution (drug solution) of a concentration of 10 mg/mL, which was stirred with heating at 65°C for 60 minutes, whereby drug introduction was performed. After the drug introduction, the liposomes were immediately cooled with ice. Subsequently, removal of the unencapsulated drug was also conducted in the same manner as in Preparation Examples 1 to 4.

As a result, as shown in Table 1, it was made clear that in the case of using bupivacaine hydrochloride, it is possible to obtain a comparatively high drug encapsulation amount, like in the case of the donepezil liposome. From these results, it was verified that bupivacaine hydrochloride can also be introduced by the pH gradient method into the liposome having the structure of FIG. 1.

### <Drug Dynamics in Bupivacaine Hydrochloride Liposome>

For the bupivacaine hydrochloride liposome prepared in Preparation Example 12 and bupivacaine hydrochloride used alone, a drug dynamics test was conducted. Subcutaneous administration into a back part of a rat was conducted in a dose, in terms of the amount of bupivacaine hydrochloride, as set forth in Table 7. After lapses of 1, 24, 72, 120, and 168 hours from the administration of the bupivacaine hydrochloride liposome composition, and after lapses of 0.5, 4, and 24 hours from the administration of the bupivacaine hydrochloride used alone, back region subcutaneous tissue in the administration site was sampled and subjected to a homogenizing treatment. Subsequently, the homogenized solution was treated, the resultant sample solution was subjected to high performance liquid chromatography determination (UV-visible absorptiometer; measurement wavelength 210 nm), and the concentration of bupivacaine hydrochloride remaining in the back region subcutaneous tissue in the administration site was determined. The results are shown in FIG. 5. The retention rate of bupivacaine hydrochloride (used alone) in the administration site was lowered to below 1% in four hours after the administration. From this result, it was verified that bupivacaine hydrochloride used alone disappears from the administration site in several hours, and it was suggested that a sustained in-blood concentration cannot be attained. On the other hand, the bupivacaine hydrochloride liposome gave a profile of sustained losing from the administration site, and about 35% of bupivacaine hydrochloride remained on the seventh day from the administration. From these results, it was suggested that the liposome administered releases bupivacaine hydrochloride in the administration site in a sustained manner. From the foregoing, it is verified that the bupivacaine hydrochloride liposome obtained according to the present invention has a long-term sustained release ability of not less than one week.

**[Table 7]**

| Preparation | Dose | Results of pharmacokinetic profile |
|---|---|---|
| Bupivacaine hydrochloride alone, subcutaneous administration | SC 5 mg/kg | FIG. 5 |
| Preparation Example 12 | SC 5 mg/kg | |

### <Preparation of Ropivacaine Hydrochloride Liposome according to Present Invention>

### (Preparation Example 14)

A liposome composition was produced in the same manner as in Preparation Example 2, except that ropivacaine hydrochloride was used as the drug, to obtain a ropivacaine hydrochloride liposome.

As a result, as shown in Table 1, it was made clear that also in the case of using ropivacaine hydrochloride, it is possible to introduce the drug by the pH gradient method and to obtain a liposome composition having a comparatively high drug encapsulation amount, in the same manner as above. Besides, also as to in-vitro release properties, there was exhibited a release profile comparable to those in the cases of the donepezil hydrochloride liposome and the bupivacaine hydrochloride liposome having shown sustained release properties. This suggests that the use of ropivacaine hydrochloride provides release performance in the same manner as in the cases of donepezil hydrochloride and bupivacaine hydrochloride.

### <Preparation of Tramadol Hydrochloride Liposome according to Present Invention>

### (Preparation Example 15)

A liposome composition was produced in the same manner as in Preparation Example 3, except that tramadol hydrochloride was used as the drug, to obtain a tramadol hydrochloride liposome.

As a result, as shown in Table 1, it was verified that also in the case of using tramadol hydrochloride, it is possible to introduce the drug by the pH gradient method and to obtain a liposome composition having a comparatively high drug encapsulation amount, in the same manner as above.

## Claims

1. A liposome composition comprising:
a first liposome having an outer membrane comprised of a multilayered lipid bilayer; and
a plurality of second liposomes accommodated in a first liposome inner region defined by the outer membrane, the second liposomes each having an outer membrane comprised of a multilayered lipid bilayer,
wherein the liposome composition has second liposome inner regions each defined by the outer membrane of each of the second liposomes, and
an ion gradient is formed at least between each of the second liposome inner regions and the outside of the first liposome,
wherein lipid membranes of the first liposome and the second liposomes are each comprised of lipid including phospholipid and cholesterol, and
wherein the liposome composition is prepared as defined in claim 11.

2. The liposome composition according to claim 1,
wherein the ion gradient is proton concentration gradient, and pH in the second liposome inner region or pH in the second liposome inner region and the first liposome inner region is lower than pH in the outside of the first liposome.

3. The liposome composition according to claim 1 or 2, wherein the first liposome has an average particle diameter within a range of 1 to 20 µm.

4. The liposome composition according to any of claims 1 to 3, wherein a drug is contained in the second liposome inner region or in the second liposome and first liposome inner regions.

5. The liposome composition according to claim 4,
wherein the drug is contained in a molar ratio (mol/mol) of not less than 0.05, based on total lipid.

6. The liposome composition according to any of claims 1 to 4, wherein the drug to be encapsulated is capable of being encapsulated into liposomes by an ion gradient method, preferably is an ionizable amphipathic drug.

7. The liposome composition according to any of claims 4 to 6, wherein the drug is a drug for treatment of cerebral vascular disorder, Parkinson's disease, dementia, analgesic agents, local anesthetics, and anti-malignancy agents.

8. The liposome composition according to any of the previous claims, wherein the phospholipid is a saturated phospholipid.

9. The liposome composition according to any of the previous claims for use in treatment by subcutaneous, intramuscular, intraperitoneal, intrathecal, extradural or intraventricular administration, preferably for subcutaneous administration.

10. The liposome composition according to any of the previous claims for use in treatment by being administered by use of a syringe or a spray-type device, or by an administration being carried out through a catheter inserted in a living body.

11. A process for producing a liposome composition provided with an ion gradient between the inside and the outside of an outer membrane,
the process comprising the steps of:
mixing a first inner aqueous phase solution containing a compound for forming the ion gradient with a lipid-containing water-miscible solvent in a volume ratio of from 0.7 to 2.5 so as to prepare a first emulsion, the lipid including phospholipid and cholesterol;
mixing a second inner aqueous phase solution with the first emulsion in a volume ratio of from 0.7 to 2.5 so as to prepare a second emulsion; and
replacing an outer aqueous phase of the second emulsion with an aqueous solution which is lower than the first inner aqueous phase solution in the concentration of the compound for forming the ion gradient.

12. The process for producing a liposome composition according to claim 11, wherein the ion gradient is proton concentration gradient.

13. The process for producing a liposome composition according to claim 11 or 12, in which the first inner aqueous phase solution contains a sulfate, preferably wherein the sulfate is ammonium sulfate.

14. The process for producing a liposome composition according to any one of claims 11 to 13, further comprising
a step of introducing a drug into the inside of the liposome composition by a driving force due to the ion gradient.

## Patentansprüche

1. Liposom-Zusammensetzung umfassend:
ein erstes Liposom mit einer äußeren Membran, zusammengesetzt aus einer vielschichtigen Lipid-Doppelschicht; und
einer Vielzahl von zweiten Liposomen, die in einem inneren Bereich eines ersten Liposoms, definiert durch die äußere Membran, untergebracht sind,
die zweiten Liposomen jeweils eine äußere Membran haben, zusammengesetzt aus einer vielschichtigen Lipid-Doppelschicht,
wobei die Liposom-Zusammensetzung zweite Liposom-innere Bereiche hat, jeder definiert durch die äußere Membran von jedem dieser zweiten Liposomen, und
ein Ionen-Gradient zumindest zwischen jedem dieser zweiten Liposom-inneren Bereiche und dem Äußeren des ersten Liposoms gebildet ist,
wobei Lipid-Membranen des ersten Liposoms und der zweiten Liposome jeweils aus Lipid, einschließlich Phospholipid und Cholesterol, zusammengesetzt sind, und
wobei die Liposom-Zusammensetzung wie in Anspruch 11 definiert hergestellt ist.

2. Liposom-Zusammensetzung gemäß Anspruch 1, wobei der Ionen-Gradient ein Proton-Konzentrations-Gradient ist, und der pH in dem zweiten Liposom-inneren Bereich oder der pH in dem zweiten Liposom-inneren Bereich und dem ersten Liposom-inneren Bereich niedriger ist als der pH in dem Äußeren des ersten Liposoms.

3. Liposom-Zusammensetzung gemäß Anspruch 1 oder 2, wobei das erste Liposom einen durchschnittlichen PartikelDurchmesser in einem Bereich von 1-20 µm hat.

4. Liposom-Zusammensetzung gemäß irgendeinem der Ansprüche 1 bis 3, wobei ein Wirkstoff in dem zweiten Liposom-inneren Bereich oder in den inneren Bereichen des zweiten Liposoms und des ersten Liposoms enthalten ist.

5. Liposom-Zusammensetzung gemäß Anspruch 4, wobei der Wirkstoff in einem molaren Verhältnis (mol/mol) von nicht weniger als 0,05 enthalten ist, basierend auf dem Gesamtlipid.

6. Liposom-Zusammensetzung gemäß irgendeinem der Ansprüche 1 bis 4, wobei der einzukapselnde Wirkstoff in der Lage ist, in Liposomen durch ein Ionen-Gradienten-Verfahren eingekapselt zu werden, bevorzugt ist ein ionisierbarer amphipathischer Wirkstoff.

7. Liposom-Zusammensetzung gemäß irgendeinem der Ansprüche 4 bis 6, wobei der Wirkstoff ein Wirkstoff zur Behandlung von zerebraler vaskulärer Krankheit, Parkinson's Krankheit, Demenz, ist, analgetische Agenzien, Lokalanästhetika und Anti-Malignitäts-Agenzien.

8. Liposom-Zusammensetzung gemäß irgendeinem der vorhergehenden Ansprüche, wobei das Phospholipid ein gesättigtes Phospholipid ist.

9. Liposom-Zusammensetzung gemäß irgendeinem der vorhergehenden Ansprüche zur Verwendung bei Behandlung durch subkutane, intramuskuläre, intraperitoneale, intrathekale, extradurale oder intraventrikuläre Verabreichung, bevorzugt für subkutane Verabreichung.

10. Liposom-Zusammensetzung gemäß irgendeinem der vorhergehenden Ansprüche zur Verwendung bei der Behandlung durch Verabreichung durch Verwendung einer Spritze oder einer Spray-artigen Vorrichtung, oder durch eine Verabreichung, die ausgeführt wird durch einen Katheter, der in einen lebenden Körper eingeführt wird.

11. Verfahren zur Herstellung einer Liposom-Zusammensetzung, die ausgestattet ist mit einem Ionen-Gradienten zwischen dem Inneren und dem Äußeren einer äußeren Membran, das Verfahren umfassend die Schritte des:
Mischens einer erste-innere-wässrige-Phase-Lösung enthaltend eine Verbindung zum Bilden des Ionen-Gradienten mit einem Fett-enthaltenden Wasser-mischbaren Lösungsmittel in einem Volumen-Verhältnis von 0,7 bis 2,5, um eine erste Emulsion herzustellen, das Lipid schließt Phospholipid und Cholesterol ein,
Mischens einer zweite-innere-wässrige-Phase-Lösung mit der ersten Emulsion in einem Volumen-Verhältnis von 0,7 bis 2,5, um eine zweite Emulsion herzustellen; und
Ersetzens einer äußeren wässrigen Phase der zweiten Emulsion mit einer wässrigen Lösung, welche niedriger ist als die erste-innere-wässrige-Phase-Lösung in der Konzentration der Verbindung zum Bilden des Ionen-Gradienten.

12. Verfahren zur Herstellung einer Liposom-Zusammensetzung gemäß Anspruch 11, wobei der Ionen-Gradient ein Proton-Konzentrations-Gradient ist.

13. Verfahren zur Herstellung einer Liposom-Zusammensetzung gemäß Anspruch 11 oder 12, in welcher die erste-innere-wässrige-Phase-Lösung ein Sulfat enthält, bevorzugt wobei das Sulfat Ammoniumsulfat ist.

14. Verfahren zur Herstellung einer Liposom-Zusammensetzung gemäß irgendeinem der Ansprüche 11 bis 13, ferner umfassend
einen Schritt des Einführens eines Wirkstoffs in das Innere der Liposom-Zusammensetzung durch eine treibende Kraft aufgrund des Ionen-Gradienten.

## Revendications

1. Composition de liposomes renfermant :
un premier liposome ayant une membrane externe constituée d'une double couche lipidique stratifiée, et
un ensemble de seconds liposomes logés dans la région interne du premier liposome définie par la membrane externe, les seconds liposomes ayant chacun une membrane externe constituée par une double couche lipidique stratifiée,
la composition de liposomes ayant des secondes régions internes de liposome définies chacune par la membrane externe de chacun des seconds liposomes, et
un gradient ionique étant formé au moins entre chacune des secondes régions internes de liposome et l'extérieur du premier liposome,
les membranes lipidiques du premier liposome et des seconds liposomes étant chacune constituées de lipides renfermant un phospholipide et du cholestérol, et
la composition de liposomes étant obtenue conformément à la revendication 11.

2. Composition de liposomes conforme à la revendication 1,
dans laquelle le gradient ionique est un gradient de concentration de protons et le pH dans la seconde région interne de liposome ou le pH dans la seconde région interne de liposome et la première région interne de liposome est inférieur au pH à l'extérieur du premier liposome.

3. Composition de liposomes conforme à la revendication 1 ou 2,
dans laquelle le premier liposome a un diamètre de particule moyen situé dans la plage de 1 à 20 µm.

4. Composition de liposomes conforme à l'une quelconque des revendications 1 à 3,
dans laquelle un médicament est renfermé dans la seconde région interne de liposome ou dans la seconde et dans la première régions internes de liposome.

5. Composition de liposomes conforme à la revendication 4,
dans laquelle le médicament est renfermé selon un rapport molaire (mol/mol) non inférieur à 0,05 par rapport à la quantité totale de lipides.

6. Composition de liposomes conforme à l'une quelconque des revendications 1 à 4,
dans laquelle le médicament destiné à être encapsulé est susceptible d'être encapsulé dans des liposomes par un procédé de gradient ionique, et est de préférence un médicament amphipathique ionisable.

7. Composition de liposomes conforme à l'une quelconque des revendications 4 à 6,
dans laquelle le médicament est un médicament destiné au traitement d'affections vasculaires cérébrales, de la maladie de Parkinson, de la démence, un agent analgésique, un anesthésique local ou un agent anticancéreux.

8. Composition de liposomes conforme à l'une quelconque des revendications précédentes,
dans laquelle le phospholipide est un phospholipide saturé.

9. Composition de liposomes conforme à l'une quelconque des revendications précédentes,
destinée à être administré par voie sous-cutanée, intramusculaire, intrapéritonéale, intrathécale, extradurale ou intraventriculaire, de préférence par voie sous-cutanée.

10. Composition de liposomes conforme à l'une quelconque des revendications précédentes,
destinée à être administrée en utilisant une seringue ou un dispositif de type pulvérisateur, ou par l'intermédiaire d'un cathéter inséré dans un corps vivant.

11. Procédé d'obtention d'une composition de liposomes ayant un gradient ionique entre l'intérieur et l'extérieur d'une membrane externe, comprenant des étapes consistant à :
mélanger une première solution à phase aqueuse interne renfermant un composé destiné à former un gradient ionique avec un solvant missible à l'eau renfermant un lipide selon un rapport volumique de 0,7 à 2,5 pour permettre de préparer une première émulsion, le lipide renfermant un phospholipide et du cholestérol,
mélanger une seconde solution à phase aqueuse interne avec la première émulsion selon un rapport volumique de 0,7 à 2,5 pour préparer une seconde émulsion, et
remplacer la phase aqueuse externe de la seconde émulsion par une solution aqueuse dont la concentration en composé est inférieure à celle de la première solution à phase aqueuse interne pour former le gradient ionique.

12. Procédé d'obtention d'une composition de liposomes conforme à la revendication 11,
selon lequel le gradient ionique est un gradient de concentration de protons.

13. Procédé d'obtention d'une composition de liposomes conforme à la revendication 11 ou 12,
selon lequel la première solution à phase aqueuse interne renferme un sulfate, le sulfate étant de préférence du sulfate d'ammonium.

14. Procédé d'obtention d'une composition de liposomes conforme à l'une quelconque des revendications 11 à 13,
comprenant en outre une étape consistant à introduire un médicament à la partie interne de la composition de liposomes par une force d'entraînement provoquée par le gradient ionique.
